# NOUVEAU FASCICULE DE BREVET EUROPEEN

(11) **EP 0 275 796 B2**
(45) Date de publication et mention de la décision concernant l'opposition: **06.09.1995**
(45) Mention de la délivrance du brevet: 25.03.1992
(21) Numéro de dépôt: 87402997.8
(22) Date de dépôt: 28.12.1987
(51) Int. Cl.: B01J 13/00, A61K 9/50

(54) **Procédé de préparation de systèmes colloidaux dispersibles d'une substance, sous forme du nanoparticules**
Herstellungsverfahren für kolloid-disperse Systeme aus einer Substanz in Form von Nanopartikeln
Preparation process for disperse colloidal systems from a substance in the shape of nanoparticles

(30) Priorité: 31.12.1986 FR 8618446
(43) Date de publication de la demande: 27.07.1988
(73) Titulaire: CENTRE NATIONAL DE LA RECHERCHE SCIENTIFIQUE, 75007 Paris (FR)
(72) Inventeur: Fessi, Hatem, F-75014 Paris (FR); Devissaguet, Jean-Philippe, F-92200 Neuilly/Seine (FR); Puisieux, Francis, F-94700 Maisons Alfort (FR); Thies, Curt, Saint Louis (US)
(74) Mandataire: Polus, Camille

(56) Documents cités:
- EP-A- 0 169 618
- EP-B- 0 065 193
- WO-A-86/03676
- AU-A- 495 261
- CH-A- 201 942
- DE-C- 738 604
- FR-A- 2 515 960
- Ullmanns Encyklopädie der technischen Chemie, 3. Auflage, 10.Band, Urban u. Schwarzenberg-Verlag, München-Berlin, 1958, Seiten 605-606
- Rudolf Voigt, "Lehrbuch der pharmazeutischen Technologie, 5. Auflage, Verlag Chemie, 1984, Seiten 108-112
- D.C. Black by "High Polymer Latices", Vol. 1 - Fundamental Principles, Madaren & Sons Ltd., London - Palmerton Publishing Co.Inc., New York, 1966, Seiten 356-357

## Description

La présente invention a pour objet un nouveau procédé de préparation de systèmes colloïdaux dispersibles d'une substance sous forme de particules sphériques de type matriciel et de taille inférieure à 500 nm (nanoparticules).

Des particules submicroscopiques d'un diamètre inférieur à 500 nm sont déjà connues notamment d'après les brevets BE-A-808 034, BE-A-839 748, BE-A-869 107 et FR-A-2 504 408. Selon BE-A-808 034 et -839 748, les particules submicroscopiques sont formées par polymérisation micellaire d'un monomère tel qu'un dérivé de l'acide acrylique. De même, BE-A-869 107 et FR-A-2 504 408 décrivent la préparation de nanoparticules biodégradables obtenues par polymérisation d'un cyanoacrylate d'alkyle et contenant une substance biologiquement active. Les méthodes font appel à une polymérisation en solution et sont donc limitées à l'utilisation d'un nombre limité de polymères pouvant être obtenus notamment par vinyl-addition et ne peuvent convenir à des polymères naturels ou hémisynthétiques. De plus il est difficule de maîtriser le poids moléculaire du polymère constitutif des nanoparticules et il est nécessaire, notamment en vue d'usage biologique, d'éliminer les monomères et oligopolymères résiduels, le cas échéant les réactifs de polymérisation (initiateur et catalyseur) en excès, ainsi que les surfactifs s'ils sont utilisés à forte concentration ou ne sont pas biocompatibles. Or la purification (ultracentrifugation, dialyse) s'avère souvent lourde, car la filtration des nanoparticules, vu leur taille, n'est pas toujours possible.

On a aussi décrit des méthodes par émulsification-évaporation, faisant appel à des polymères préformés et selon lesquelles une solution organique de polymère non miscible à l'eau est émulsifiée dans une phase continue aqueuse, puis le solvant est évaporé afin de conduire à une suspension de polymère insoluble dans l'eau. Cependant, si l'intérêt majeur de cette méthode est d'être utilisable pour de nombreux polymères d'origine synthétique ou hémisynthétique et de permettre ainsi l'obtention de nanoparticules à partir de polymères bien définis, son inconvénient réside dans la difficulté de réaliser des émulsions ultrafines et homogènes pour l'obtention de nanoparticules inférieures à 500 nm qui seraient elles-mêmes de taille homogène. De plus, la nécessité d'utilisation de surfactifs dans des proportions souvent importantes (20%) et qui devront être éliminés, et la mise en oeuvre d'appareillages sophistiqués et gourmands en énergie (sonicateurs, homogénéiseurs,..) constituent des handicaps sérieux à leurs applications industrielles.

On a aussi proposé l'obtention de nanoparticules dans le cas des protéines, notamment par dénaturation à chaud d'une émulsion eau dans l'huile d'une solution protéique, telle que l'albumine, (Kramer, P. A : J. Pharm. Sci., 63, 1646 (1974), ou par désolvatation d'une solution protéique, telle que la gélatine, par un sel minéral ou l'éthanol (Marty et coll., dans Austr. J. Pharm. Sci., 6, 65 (1978), ou dans Pharm. Acta Helv. 53, Nr. 1 (-(1978)), puis, dans les deux cas, par durcissement au moyen d'un aldéhyde. La méthode de Kramer a pour inconvénient principal de nécessiter une émulsification préalable de la solution aqueuse de la matière première macromoléculaire dans une phase continue huileuse. Cette émulsion devant être très fine, l'utilisation de surfactifs et d'appareillages ad-hoc (sonicateur, etc) est indispensable pour obtenir des nanoparticules de taille convenable. Quant à la méthode de Marty, elle fait appel à des quantités importantes de sels minéraux qu'il faut éliminer, de même que l'excès d'aldéhyde et le sulfite ou le métabisulfite utilisé pour neutraliser ce dernier.

Par ailleurs, AU-A-495 261 décrit un procédé de préparation de nanoparticules de 10 à 1000 nm à base d'une matière de macromolécules naturelles réticulées, utilisant une méthode de désolvatation/fixation.

CH-A-201 942 décrit un procédé de préparation de saumures ou de suspensions aqueuses de particules de substances organiques et de protéines.

Le document FR-A-1 571 090 décrit un procédé de préparation de microcapsules de matières huileuses avec paroi polymère utilisant une méthode par dilution.

Toutes les méthodes décrites ci-dessus ne sont applicables qu'à certaines catégories de molécules et font intervenir des opérations coûteuses (ultracentrifugation, sonication, etc) ou difficilement contrôlables (polymérisation), tout en ne permettant pas d'obtenir une homogénéité satisfaisante de la taille des particules ou même des particules suffisamment petites (inférieures à 500 nm) pour assurer leur stabilité à long terme sous forme d'une suspension colloïdale.

L'invention propose un nouveau procédé de préparation de nanoparticules, ne présentant pas les désavantages précédents et utilisable aussi bien pour des substances polymèriques synthétiques et naturelles que pour diverses substances organiques (médicaments, lipides, etc) ou minérales (sels, pigments, etc), ainsi que leurs mélanges.

La présente invention a pour objet un procédé de préparation de systèmes colloïdaux dispersibles d'une substance biologiquement active et d'un polymère, sous forme de particules sphériques de type matriciel et de taille inférieure à 500 nm. (nanoparticules), caractérisé en ce que :
(1) on prépare une phase liquide constituée essentiellement par une solution de la substance dans un solvant ou dans un mélange de solvants, et pouvant être additionnée d'un ou de plusieurs surfactifs,
(2) on prépare une seconde phase liquide constituée essentiellement part un non-solvant ou un mélange de non-solvants de la substance biologiquement active et du polymère et pouvant être additionnée d'un ou de plusieurs surfactifs, le non-solvant ou le mélange de non-solvants étant miscible en toutes proportions au solvant ou mélange de solvants,
(3) on ajoute sous agitation modérée, l'une des phases liquides obtenues sous (1) ou (2) à l'autre, de manière à obtenir une suspension colloïdale de nanoparticules de la substance, biologiquement active et du polymère, et
(4) si l'on désire, on élimine tout ou partie du solvant ou du mélange de solvants et du non-solvant ou du mélange de non-solvants, de manière à obtenir une suspension colloïdale de concentration voulue en nanoparticules ou à obtenir une poudre de nanoparticules.

Dans l'étape (3), les nanoparticules se forment pratiquement instantanément. La solution devient blanche, laiteuse et présente l'effet Tyndall caractéristique des suspensions colloïdales. Dans cette étape, on ajoute de préférence la phase liquide obtenue dans l'étape (1) à la phase liquide obtenue dans l'étape (2), notamment si celle-ci est aqueuse mais l'inverse est toutefois possible comme le montrent les exemples.

Le polymere peut être en particulier aussi bien un polymère synthétique, par exemple l'acide poly (d,1) lactique (PLA), etc, un polymère hémisynthétique comme par exemple l'acétate butyrate de cellulose, l'éthylcellulose, le phtalate d'hydroxyméthyl-propylcellulose (HPMCP), etc, qu'un polymère naturel, par exemple la gélatine, la gomme arabique, etc. De nombreux autres polymères peuvent être utilisés, par exemple : l'acéto-phtalate de polyvinyle, l'acétophthalate de cellulose; des dérivés maléiques (par ex. "Gantrez"®); les copolymères d'acide acrylique et d'acrylates et les polymères acryliques (par ex. Eudragit®); l'acide polylactique d ou l, et (d,l): les copolymères d'acide lactique et d'acide glycolique, polypeptides, glycolides (dérivés de propiolactone, butyrolactone, pivalolactone ε -caprolactone, etc); les polymères obtenus à partir d'esters cycliques des acides hydroxybutyrique, hydroxyisobutyrique, hydroxyméthylvalérique, phényl-lactique, hydroxyéthylbutyrique; le poly béta malate de benzyle; les copolymères de l'acide malique et du malate de benzyle; un copolymère polyvinylpyrrolidone-acétate de vinyle réticulé, les polycyanoacrylates d'alkyle; les poly (éthylène-vinylacétate); les polymères hydrosolubles (gélatine, gomme arabique, méthylcellulose, etc); les oligomères (styrène allyl alcool).

La substance biologiquement active peut être, notamment un principe actif médicamenteux ou un précurseur de principe actif médicamenteux, ou encore un produit de contraste ou un réactif biologique.

La "substance" peut aussi être un pigment une encre, un lubrifiant, un agent de traitement de surface, etc.

Il est évident que le procédé selon l'invention peut s'appliquer aussi bien pour une substance que pour plusieurs.

A cet égard, selon une variante du procédé, on peut fixer une seconde substance par adsorption à la surface de nanoparticules déjà formées dans l'étape (3), par simple addition à la suspension colloïdale du polymère, éventuellement après concentration. Cette seconde substance peut être en particulier une substance biologiquement active.

Le "solvant" ou le mélange de solvants utilisé est un liquide capable de dissoudre le polymère et la substance biologiquement active. Par ailleurs le solvant doit être miscible en toutes proportions avec le non-solvant. Ainsi dans la plupart des cas, le solvant sera organique de sorte que la phase liquide (1) constituera la phase organique tandis que la phase liquide (2) pourra constituer la phase aqueuse, mais il est possible d'utiliser soit deux phases organiques soit deux phases aqueuses dans la mesure où les conditions de solubilité, d'insolubilité et de miscibilité sont remplies. D'un autre côté, le solvant devra être suffisamment volatil pour pouvoir éventuellement être éliminé. Le solvant peut être choisi parmi un alcool inférieur (méthanol, éthanol, isopropanol), une cétone inférieure (acétone, méthyl-éthyl-cétone), un hydrocarbure léger ou un `mélange d'hydrocarbures légers (hexane, éther de pétrole) un hydrocarbure léger chloré (chloroforme, chlorure de méthylène, trichloréthylène, etc), ou d'autres solvants légers usuels tels que l'acétonitrile, le dioxanne.

Le "non-solvant", ou le mélange de non-solvants de la substance est un liquide ne dissolvant pas la substance tout en étant miscible en toutes proportions au solvant utilisé. Ainsi, par exemple, lorsque la substance est un polymère tel que P.L.A., le solvant peut être l'acétone et le non-solvant peut être l'éthanol ou l'eau distillée; si la substance est par exemple un polymère acrylique tel que Eudragit L100® , le solvant peut être une phase aqueuse alcaline et le non-solvant peut être une phase aqueuse acide. Il peut être avantageux d'ajouter au solvant une faible proportion (inférieure à 20% en volume, par exemple environ 10% en vol.) de non-solvant, dans la phase liquide (1), ce qui permet d'obtenir des nanoparticules plus petites, notamment inférieures à 100 nm.

Il est souhaitable, pour obtenir une suspension plus stable, d'ajouter un ou plusieurs surfactifs (ou émulsifiants). Les surfactifs peuvent être anioniques (par exemple laurylsulfate de sodium), cationiques (par exemple ammonium quaternaire) ou non ioniques (par exemple monoesters de sorbitanne polyoxyéthylénés ou non, éthers d'alcools gras et de polyoxyéthylène-glycols, polyoxyéthylène-polypropylène glycol).

Toutefois, les nanoparticules peuvent être obtenues selon l'invention sans surfactifs, et ceux-ci ne sont d'ailleurs pas nécessaires si dans l'étape (4) la totalité des solvants et des non-solvants est éliminée, par exemple par lyophilisation. On peut ainsi obtenir des nanoparticules lyophilisées dont la conservation est de longue durée.

La proportion en surfactifs dans la suspension colloïdale obtenue dans l'étape (3) lorsqu'ils sont présents, peut aller notamment de 0,1% à 10% en poids, et de préférence entre 0,2 et 2% en poids.

Dans le cas ou la substance est un polymère, la concentration du polymère dans le solvant ou le mélange de solvants peut être comprise entre 0,1 et 10%, de préférence entre 0,2 et 2% en poids.

Le rapport de volumes des solvants et des non-solvants doit être tel qu'il puisse permettre la précipitation du polymère. Lorsque ce rapport augmente, la taille des nanoparticules diminue.

L'agitation modérée de la préparation dans l'étape (3) est liée à la quantité de produits mise en oeuvre. Elle n'est pas nécessaire pour de faibles quantités.

L'influence de la température et de pH sont limitées dans le procédé selon l'invention, de sorte qu'il n'est généralement pas nécessaire d'opérer dans des conditions particulières. Toutefois, lorsqu'on utilise deux phases aqueuses (1) et (2), leurs pH respectifs devront être différents, pour remplir respectivement les conditions de solvant et de non-solvant.

Par ailleurs, la présence d'électrolyte (par exemple le chlorure de sodium) ne semble pas avoir d'influence sur l'obtention des nanoparticules. Ainsi après formation des nanoparticules dans l'exemple 1, une concentration de 25 mg/ml de chlorure de sodium n'entraîne pas de coalescence ou de précipitation des nanoparticules formées.

Les particules obtenues selon l'invention sont autoclavables si les propriétés physiques de la substance le permettent.

Le procédé d'obtention de nanoparticules selon l'invention apporte les avantages suivants par rapport aux procédés connus:
- obtention de nanoparticules inférieures à 500 nm et notamment d'environ 200 nm par une méthode simple ne nécessitant pas un grand apport d'énergie;
- dans le cas où la substance comprend un polymère, les nanoparticules ne sont plus obtenues par polymérisation d'un monomère mais par "nanoprécipitation" d'un polymère bien défini;
- utilisation de polymères naturels aussi bien que synthétiques dont l'innocuité est reconnue et l'application dans le domaine de la santé est très ancienne;
- utilisation de polymères qui, selon leur nature, peuvent être biocompatibles;
- possibilité d'utilisation de polymères qui peuvent se dissoudre à partir d'un certain pH dans l'organisme, d'où l'assurance d'une non-concentration dans l'organisme de particules de polymères;
- possibilité d'utilisation de polymères qui, selon leur nature peuvent être biorésorbables, les produits de dégradation étant d'une totale inocuité;
- obtention de particules sphériques avec une faible dispersion des tailles.

Les exemples 6 et 7 suivants illustrent l'invention, les autres exemples, illustrent la preparation de nanoparticules de polymère en l'absence de la substance biologiquement active, n'illustrent pas l'invention telle que revendiqué. Les nanoparticules obtenues sont visibles au microscope électronique à transmission (x 25000-150000) et se présentent, après coloration négative avec l'acide phosphotungstique, sous la forme de particules non contrastées sensiblement rondes.

### Exemple 1 : Préparation de nanoparticules de polymères.

On dissout d'une part, 125 mg d'acide poly (d,l) lactique (P.L.A.) dans 25 ml d'acétone et, d'autre part, 125 mg de polymère mixte d'oxyde d'éthylène et de propylène-glycol (Pluronic F68 ® ou Poloxamer 188®), agent de surface non ionique, dans 50 ml d'eau purifiée.

On ajoute, sous agitation magnétique (100 r.p.m.), la phase acétonique à la phase aqueuse. Le milieu devient immédiatement opalescent par formation de nanoparticules du polymère (P.L.A.). La taille moyenne des nanoparticules, mesurée aussitôt après préparation dans un diffractomètre à rayon laser (Nanosizer® de la firme Coultronics), est de 200 nm avec un indice moyen de dispersion de 0,5.

L'acétone est éliminée sous pression réduite (vide de la trompe à eau), puis la suspension est concentrée dans ces mêmes conditions jusqu'au volume désiré, par exemple : 10 ml.

La suspension concentrée de nanoparticules est filtrée sur un verre fritté (pores 9-15 µm) ou sur une membrane filtrante (pores 5 µm), et la taille des nanoparticules, à nouveau mesurée dans le filtrat, demeure inchangée ainsi que l'indice de dispersion. L'examen en microscopie électronique à transmission montre l'aspect sphérique régulier des nanoparticules d'acide (d,l) polylactique.

Après un repos prolongé (18 mois), l'aspect de la suspension de nanoparticules demeure inchangé et on n'observe, en particulier, aucune sédimentation irréversible, ni variation de la taille des nanoparticules.

### Exemple 2 (variante de l'exemple 1)

On procède comme dans l'exemple 1, mais en ajoutant la phase aqueuse à la phase acétonique. Les nanoparticules obtenues présentent les mêmes caractéristiques qu dans l'exemple 1.

### Exemple 3 (variante de l'exemple 1)

On procède comme dans l'exemple 1, mais en ajoutant la phase acétonique à la phase aqueuse sans agitation du milieu. Les nanoparticules obtenues ont une taille moyenne de 205 nm avec un indice moyen de dispersion de 1.

### Exemple 4 (variante de l'exemple 1)

On procède comme dans l'exemple 1, mais sans ajouter d'agent de surface à la phase aqueuse. Les nanoparticules obtenues ont une taille moyenne de 207 nm, avec un indice moyen de dispersion de 1,3.

### Exemple 5: Préparation de nanoparticules contenant de l'indométacine (principe actif lipophile)

a) On procède comme dans l'exemple 1, mais on ajoute 5 mg d'indométacine dans la phase acétonique. Les nanoparticules obtenues ont une taille moyenne de 180 nm avec un indice moyen de dispersion de 1,5. Après ultracentrifugation et dosage de l'indométacine dans la phase dispersante, la quantité de principe actif incorporé dans les nanoparticules représente 80% de la quantité initiale.
b) Essai pharmacologique :
   Administrée par voie orale chez le rat à jeun (5 mg/kg d'indométacine) la suspension de nanoparticules conduit à une absorption digestive de l'indométacine plus rapide et plus complète que celle observée après administration de la même dose d'indométacine en solution. Après administration répétée chez le rat à jeun (5mg/kg d'indométacine, 3 jours de suite), la suspension de nanoparticules conduit à une meilleure tolérance digestive, objectivée par le nombre des ulcérations et hémorragies, que celle observée après administration de la même dose d'indométacine en solution.

Administrée par voie intraveineuse chez le rat (5 mg/kg d'indométacine) la suspension de nanoparticules conduit à un profil chronologique des concentrations plasmatiques d'indométacine mettant en évidence, par rapport à l'injection d'indométacine en solution, une distribution extravasculaire plus importante du principe actif (majoration d'un facteur voisin de 2 du volume de distribution de l'indométacine), suivie d'une élimination plus lente (majoration d'un facteur voisin de 2 de la demi-vie biologique de l'in dométacine).

### Exemple 6: Préparation de nanoparticules contenant de la doxorubicine (principe actif hydrophile)

a) On procède comme dans l'exemple 1, mais on ajoute 12,5 mg de doxorubicine dans la phase aqueuse. Les nanoparticules obtenues ont une taille moyenne de 288 nm et un indice moyen de dispersion de 2. Après ultracentrifugation et dosage de la doxorubicine dans la phase dispersante, la quantité de principe actif incorporé dans les nanoparticules représente 50% de la quantité initiale.
b) Essai pharmacologique : Administrée à la dose de 10 mg/kg pendant trois jours chez le rat, la suspension de nanoparticules de doxorubicine a montré une nette amélioration de la toxicité cardiaque du principe actif par rapport à son administration en solution, résultats comparables à ceux observés par COUVREUR et coll. (J. Pharm. Sci., 71, p. 790, (1982) avec des nanoparticules préparées par polymérisation d'isobutylcyanoacrylate.

### Exemple 7 : Fixation d'un principe actif (doxorubicine) sur des nanoparticules de polymère.

On procède comme dans l'exemple 1. Puis, on ajoute à la suspension de nanoparticules de P.L.A., concentrée sous un volume de 10 ml, 12,5 mg de doxorubicine. Après 72 h. de contact, la taille moyenne des nanoparticules est de 220 nm avec un indice moyen de dispersion de 2. Après ultracentrifugation et dosage de la doxorubicine dans la phase dispersante, la quantité de principe actif fixé sur les nanoparticules représente 32% de la quantité initiale.

### Exemple 8: Addition de non-solvant à la phase du solvant.

On procède comme dans l'exemple 1, mais le polymère est dissous dans un mélange acétone/eau (90/10, v/v), au lieu d'acétone pure. La présence d'une faible proportion de non-solvant du polymère dans son solvant, conduit à des nanoparticules dont la taille moyenne est de 90 nm avec un indice moyen de dispersion de 1,5.

### Exemple 9: Utilisation de deux phases aqueuses.

a) D'une part, 625 mg d'un polymère acrylique (Eudragit L 100®) sont dissous dans 125 ml d'eau purifiée additionnée de 3,45 ml. de soude 0,1 N.
   D'autre part, 625 mg de polymère mixte d'oxyde d'éthylène et de propylène glycol. (Pluronic F68 ®) sont dissous dans 250 ml d'eau purifiée additionnée de 0,85 ml d'acide acétique pur.
   La phase aqueuse polymérique basique est ajoutée, sous agitation magnétique à la phase aqueuse acide. Les nanoparticules d'Eudragit L100® se forment immédiatement, rendant le milieu opalescent. Après concentration de la suspension sous pression réduite, la taille moyenne des nanoparticules est de 130 nm avec un indice moyen de dispersion de 2,3.
b) Préparation de comprimés. La suspension de nanoparticules d'Eudragit L 100 ® obtenue ci-dessus a été utilisée pour préparer une formulation d'enrobage de comprimés par pulvérisation. Les comprimés enrobés ont montré qu'ils étaient gastro-résistants pendant au moins 2 h, à pH acide (milieu gastrique de la Pharmacopée US) mais libéraient leur principe actif à pH neutre (milieu intestinal de la Pharmacopée US).

### Exemple 10 : Utilisation de deux phases organiques polaires.

D'une part, 125 mg d'acide poly (d,l) lactique sont dissous dans 25 ml de tétrahydrofuranne.

D'autre part, 125 mg de polymère mixte d'éthylène et de propylèneglycol (Pluronic F 68 ®) sont dissous dans 50 ml d'éthanol absolu.

La phase polymérique est ajoutée, sous agitation magnétique, à la phase éthanolique. Les nanoparticules d'acide poly (d,l) lactique se forment immédiatement, rendant le milieu opalescent. Après concentration de la suspension, sous pression réduite et à faible température, jusqu'à un volume de 4 ml, suivie d'une filtration sur verre fritté (pores 9-15 µm), la taille moyenne des nanoparticules est de 201 nm avec un indice moyen de dispersion de 1,6.

### Exemple 11: Utilisation de deux phases organiques apolaires.

a) D'une part, 125 mg de polymère acrylique (Eudragit L100 ®) sont dissous dans 25 ml de chloroforme.
   D'autre part, 0,2 ml de mono-oléeate de sorbitanne, (SPAN 80 ®, agent de surface non ionique) sont dissous dans 50 ml d'heptane.
   On ajoute, sous agitation magnétique, la phase chloroformique à la phase heptanique. Les nanoparticules d'Eudragit L100® se forment immédiatement, rendant le milieu opalescent.
   Après concentration de la suspension sous un volume de 30 ml, les nanoparticules ont une taille moyenne de 350 nm avec un indice moyen de dispersion de 1.
b) Préparation de comprimés.
   Utilisée dans les mêmes conditions que dans l'exemple 9b, la suspension de nanoparticules d'Eudragit L100 ® a permis la fabrication, par enrobage, de comprimés gastro-résistants.

### Exemple 12 : préparation de nanoparticules de lipide.

D'une part, 125 mg de stéarate de glycérol sont dissous dans un mélange acétone/tétrahydrofuranne (90/10, v/v).

D'autre part, 0.25 ml de mono-oléate de sorbitanne polyoxyéthyléné (TWEEN 80®, agent de surface non ionique) sont dissous dans 50 ml d'eau purifiée.

On ajoute, sous agitation magnétique, la phase organique lipidique à la phase aqueuse. Les nanoparticules de stéarate de glycérol se forment immédiatement, rendant le milieu opalescent.

Après concentration de la suspension sous un volume de 10 ml, les nanoparticules ont une taille moyenne de 300 nm avec un indice moyen de dispersion de 3.

### Exemple 13 (variante de l'exemple 12)

D'une part, 125 mg de palmito-stéarate de glycérol et 0,1 ml de mono-oléate de sorbitanne (SPAN 80®) sont dissous dans 50 ml d'éthanol absolu.

D'autre part, 0,1 ml de mono-oléate de sorbitanne polyoxythyléné (TWEEN 80®) sont dissous dans 50 ml d'eau purifiée.

On ajoute, sous agitation magnétique, la phase alcoolique lipidique à la phase aqueuse. Les nanoparticules de palmito-stéarate de glycérol se forment instantanément, rendant le milieu opalescent.

Après concentration de la suspension sous un volume de 10 ml, les nanoparticules ont une taille moyenne de 160 nm avec un indice moyen de dispersion de 2.

### Exemple 14 : Préparation de nanoparticules d'indométacine.

a) On procède comme indiqué dans l'exemple 1, mais en remplaçant le polymère par 25 mg d'indométacine.
   Après concentration de la suspension par évaporation du solvant organique, les nanoparticules d'indométacine ont une taille moyenne de290 nm et un indice moyen de dispersion de 2. L'examen en microscopie électronique à transmission montre l'aspect sphérique et non cristallin des nanoparticules d'indométacine.
b) Essai pharmacologique
   Admistrée par voie orale chez le rat à jeun (5 mg/kg) la suspension de nanoparticules d'indométacine conduit à une absorption plus rapide et plus complète que celle observée par administration de doses égales d'indométacine en solution.

Administrée par voie intraveineuse chez le rat (5 mg/kg) la suspension de nanoparticules d'indométacine conduit à un profil chronologique des concentrations plasmatiques d'indométacine mettant en évidence, par rapport à l'injection de doses égales d'indométacine en solution, une distribution extravasculaire plus importante du principe actif (majoration du volume de distribution), suivie d'une élimination plus lente (majoration de la demi-vie biologique).

### Exemple 15 : Lyophilisation des nanoparticules de polymère

On procède comme indiqué dans l'exemple 1. Après concentration de la suspension de nanoparticules de P.L.A. jusqu'à un volume de 20 ml, on ajoute à celle-ci 200 mg de tréhalose, puis on lyophilise.

Après dispersion du lyophilisat dans 10 ml d'eau purifiée, les nanoparticules ont une taille moyenne de 275 nm avec un indice moyen de dispersion de 1,5.

### Exemple 16 : Stabilité des nanoparticules de polymère en présence de forces ioniques variables.

On procède comme indiqué dans l'exemple 1. Après concentration de la suspension de nanoparticules de P.L.A. jusqu'à un volume de 10 ml, on ajoute progressivement à celle-ci des quantités croissantes de chlorure de sodium. La suspension de nanoparticules est parfaitement stable lorsque la concentration en chlorure de sodium correspond à l'isotonie avec le sang, et le demeure jusqu'à des concentrations en chlorure de sodium supérieure à 3 fois la concentration isotonique.

### Exemple 17 : Préparation de nanoparticules en présence d'un sel.

On procède comme indiqué dans l'exemple 1, mais la phase aqueuse est additionnée de 90 mg de chlorure de sodium. Après concentration de la suspension de nanoparticules jusqu'à un volume de 10 ml, correspondant compte tenu du chlorure de sodium à l'isotonie avec le sang, les nanoparticules ont une taille moyenne de 250 nm avec un indice moyen de dispersion de 2.

La suspension demeure stable dans le temps et ne présente, après 12 mois de conservation, ni sédimentation irréversible, ni variation de la taille des nanoparticules.

Les nanoparticules obtenues selon l'invention peuvent trouver des applications dans de nombreux secteurs techniques.

En tant que "vecteurs" de médicaments, en thérapeutique humaine et animale les nanoparticules permettent d'envisager :
- d'atteindre de nouveaux sites d'action, en particulier intracellulaires, voire intralysosomiaux;
- d'utiliser de nouvelles voies d'administration pour des médicaments connus, en augmentant la stabilité et/ou l'absorption des médicaments, ou en permettant la réalisation de formes injectables, par voie intravasculaire, de médicaments insolubles ;
- de modifier la distribution tissulaire des médicaments, par un meilleur ciblage vers les sites d'action favorables et/ou un détournement des sites d'effets indésirables voire toxiques (amélioration de l'index thérapeutique).

En pharmacie, les dispersions colloïdales de nanoparticules peuvent permettre :
- de réaliser des formes injectables de médicaments insolubles,
- de stabiliser un principe actif médicamenteux, et
- de réaliser des enrobages de formes galéniques à partir de dispersions aqueuses de polymères filmogènes.

Dans le domaine de l'agrochimie les nanoparticules peuvent véhiculer des insecticides, des pesticides etc... Leur taille peut permettre d'envisager une action plus puissante par une meilleure pénétration à travers la cuticule. La faible viscosité de la dispersion autorise une pulvérisation très facile sous forme de gouttelettes de très petite taille, plus efficaces car plus couvrantes.

Dans le domaine des peintures, vernis, et traitement des surfaces d'une manière générale, les nanoparticules permettent de véhiculer des pigments, des réactifs, des décapants sous forme de dispersion aqueuse de très faible viscosité, aisés à pulvériser ou à appliquer, et qui peut, si nécessaire, être rendue visqueuse, voir adhésive (remise en suspension des nanoparticules dans un véhicule approprié). La taille réduite des nanoparticules conduit à une très grande finesse du dépôt et à une très grande homogénéité, par exemple de pigmentation.

Les nanoparticules obtenues selon l'invention peuvent aussi être utilisées dans les domaines de. l'imprimerie et de la reprographie, du traitement de surface des textiles et fibres, de la photographie, de la lubrification.

## Revendications

1. Procédé de préparation de systèmes colloïdaux dispersibles d'une substance biologiquement active, sous forme de particules sphériques de type matriciel et de taille inférieure à 500 nm (nanoparticules), caractérisé en ce que :
(1) on prépare une phase liquide constituée essentiellement par une solution de la substance biologiquement active et d'un polymère dans un solvant ou dans un mélange de solvants, et pouvant être additionnée d'un ou de plusieurs surfactifs,
(2) on prépare une seconde phase liquide constituée essentiellement par un non-solvant ou un mélange de non-solvants de la substance biologiquement active et du polymère, pouvant être additionnée d'un ou de plusieurs surfactifs, le non-solvant ou le mélange de non-solvants étant miscible en toutes proportions au solvant ou mélange de solvants,
(3) on ajoute, sous agitation modérée, l'une des phases liquides obtenues sous (1) ou (2) à l'autre, de manière à obtenir pratiquement instantanément une suspension colloïdale de nanoparticules formées de la substance biologiquement active et du polymère,
(4) si l'on désire, on élimine tout ou partie du solvant ou du mélange de solvants et du non-solvant ou du mélange de non-solvants de manière à obtenir une suspension colloïdale de concentration voulue en nanoparticules ou à obtenir une poudre de nanoparticules.

2. Procédé selon la revendication 1, caractérisé en ce que l'on ajoute dans l'étape (3) la phase liquide obtenue dans l'étape (1) à la phase liquide obtenue dans l 'étape (2).

3. Procédé selon la revendication 1 ou la revendication 2, caractérisé en ce que l'on ajoute au solvant, dans l'étape (1), une faible proportion de non-solvant.

4. Procédé selon l'une quelconque des revendications 1 à 3, caractérisé en ce que le(s) surfactif(s) sont présents en une proportion de 0,1 à 10 % en poids de la suspension colloïdale obtenue dans l'étape (3).

5. Procédé selon la revendication 4, caractérisé en ce que le(s) surfactif(s) sont présents en une proportion de 0,2 à 2 % en poids.

6. Procédé selon la revendication 1, caractérisé en ce que la concentration du polymère dans le solvant ou le mélange de solvants est comprise entre 0,1 et 10 % en poids.

7. Procédé selon la revendication 1, caractérisé en ce que la concentration du polymère est comprise entre 0,2 et 2 % en poids.

8. Procédé selon l'une quelconque des revendications 1 à 7, caractérisé en ce que dans l'étape (4) la totalité des solvants et des non-solvants est éliminée par lyophilisation.

9. Procédé selon l'une quelconque des revendications 1 à 8, caractérisé en ce que les nanoparticules ont une taille d'environ 200 nm.

## Claims

1. Preparation process for dispersible colloidal systems of a biologically-active substance, in the form of spherical particles of matrix type and of a size smaller than 500 nm (nanoparticles), characterized in that:
(1) a liquid phase is prepared constituted essentially by a solution of the biologically-active substance and of a polymer in a solvent or in a mixture of solvents, and being able to have one or more surfactants added to it,
(2) a second liquid phase is prepared constituted essentially by a non-solvent or a mixture of non-solvents of the biologically-active substance and of the polymer, being able to have one or more surfactants added to it, the non-solvent or the mixture of non-solvents being miscible in all proportions with the solvent or mixture of solvents,
(3) one of the liquid phases obtained in (1) or (2) is added under moderate agitation to the other, so as to obtain almost instantaneously a colloid suspension of nanoparticles consisting of the biologically-active substance and the polymer,
(4) if desired, all or part of the solvent or mixture of solvents and of the non-solvent or mixture of non-solvents are eliminated so as to obtain a colloid suspension of the desired nanoparticle concentration or so as to obtain a nanoparticle powder.

2. Process according to claim 1, characterized in that in stage (3) the liquid phase obtained in stage (1) is added to the liquid phase obtained in stage (2).

3. Process according to claim 1 or claim 2, characterized in that in stage (1) a small amount of non-solvent is added to the solvent.

4. Process according to any one of claims 1 to 3, characterized in that the surfactant(s) is(are) present in a proportion of 0.1 to 10% by weight of the colloid suspension obtained in stage (3).

5. Process according to claim 4, characterized in that the surfactant(s) is(are) present in a proportion of 0.2 to 2% by weight.

6. Process according to claim 1, characterized in that the polymer concentration in the solvent or the mixture of solvents is comprised between 0.1 and 10% by weight.

7. Process according to claim 1, characterized in that the polymer concentration is comprised between 0.2 and 2% by weight.

8. Process according to any one of claims 1 to 7, characterized in that in stage (4) all the solvents and non-solvents are eliminated by lyophilization.

9. Process according to any one of claims 1 to 8, characterized in that the nanoparticles have a size of about 200 nm.

## Patentansprüche

1. Verfahren zur Herstellung kolloid-disperser Systeme aus einer biologisch aktiven Substanz in Form kugelförmiger, matrixartiger Teilchen von unter 500 nm Größe (Nanopartikel), **dadurch gekennzeichnet**, daß:
(1) man eine flüssige Phase herstellt, welche im wesentlichen aus einer Lösung der biologisch aktiven Substanz und eines Polymers in einem Lösungsmittel oder einem Lösungsmittelgemisch besteht und welcher ein oder mehrere oberflächenaktiven Stoffe zugesetzt werden können,
(2) man eine zweite flüssige Phase herstellt, welche im wesentlichen aus einem Nichtlösungsmittel oder einem Nichtlösungsmittelgemisch der biologisch aktiven Substanz und des Polymers besteht, welcher ein oder mehrere oberflächenaktiven Stoffe zugesetzt sein können, wobei das Nichtlösungsmittel und das Nichtlösungsmittelgemisch in allen Verhältnissen mit dem Lösungsmittel oder dem Lösungsmittelgemisch für die Substanz mischbar sind,
(3) man unter mäßigem Rühren eine der in (1) oder (2) erhaltenen flüssigen Phasen der anderen zufügt, um praktisch augenblicklich eine kolloidale Suspension von Nanopartikeln zu erhalten, gebildet aus der biologisch aktiven Substanz und dem Polymer,
(4) falls gewünscht, man das gesamte oder einen Teil des Lösungsmittels oder Lösungsmittelgemisches oder des Nichtlösungsmittels oder Nichtlösungsmittelgemisches entfernt, um eine kolloidale Suspension von erwünschter Nanopartikel-Konzentration zu erhalten oder ein Nanopartikel-Pulver zu erhalten.

2. Verfahren nach Anspruch 1, dadurch gekennzeichnet, daß man in Schritt (3) die in Schritt (1) erhaltene flüssige Phase der in Schritt (2) erhaltenen flüssigen Phase zufügt.

3. Verfahren nach Anspruch 1 oder Anspruch 2, dadurch gekennzeichnet, daß man in Schritt (1) dem Lösungsmittel ein Nichtlösungsmittel in geringem Verhältnis zusetzt.

4. Verfahren nach einem der Ansprüche 1 bis 3, dadurch gekennzeichnet, daß das (die) oberflächenaktive(n) Mittel in einem Verhältnis von 0,1 bis 10 Gew.-% bezogen auf die in Schritt (3) erhaltene kolloidale Suspension vorhanden sind.

5. Verfahren nach Anspruch 4, dadurch gekennzeichnet, daß das (die) oberflächenaktive(n) Mittel in einem Verhältnis von 0,2 bis 2 Gew.-% vorhanden sind.

6. Verfahren nach Anspruch 1, dadurch gekennzeichnet, daß die Konzentration des Polymers im Lösungsmittel oder Lösungsmittelgemisch zwischen 0,1 und 10 Gew.-% liegt.

7. Verfahren nach Anspruch 1, dadurch gekennzeichnet, daß die Konzentration des Polymers zwischen 0,2 und 2 Gew.-% liegt.

8. Verfahren nach einem der Ansprüche 1 bis 7, dadurch gekennzeichnet, daß in Schritt (4) die gesamten Lösungsmittel und Nichtlösungsmittel durch Gefriertrocknung entfernt werden.

9. Verfahren nach einem der Ansprüche 1 bis 8, dadurch gekennzeichnet, daß die Nanopartikel eine Größe von etwa 200 nm haben.
